# EUROPÄISCHE PATENTSCHRIFT

(11) **EP 2 593 779 B1**
(45) Veröffentlichungstag und Bekanntmachung des Hinweises auf die Patenterteilung: **21.05.2014**
(21) Anmeldenummer: 11782363.3
(22) Anmeldetag: 07.07.2011
(51) Int. Cl.: G01N 27/12, G01N 33/00

(54) **GAS-SENSOR UND VERFAHREN ZU SEINER HERSTELLUNG**
GAS SENSOR AND METHOD FOR PRODUCING THE SAME
CAPTEUR DE GAZ ET PROCÉDÉ DE FABRICATION CORRESPONDANT

(30) Priorität: 13.07.2010 DE 102010027070
(43) Veröffentlichungstag der Anmeldung: 22.05.2013
(73) Patentinhaber: Eberhard Karls Universität Tübingen, 72074 Tübingen (DE); Universität Bremen, 28359 Bremen (DE)
(72) Erfinder: BARSAN, Nicolae, 72076 Tübingen (DE); WEIMAR, Udo, 72072 Tübingen (DE); KEMMLER, Jens, 72827 Wannweil (DE); MAEDLER, Lutz, 28355 Bremen (DE); POKHREL, Suman, 28259 Bremen (DE)
(74) Vertreter: Patentanwälte Ruff, Wilhelm, Beier, Dauster & Partner
(86) Internationale Anmeldenummer: PCT/DE2011/001417
(87) Internationale Veröffentlichungsnummer: WO 2012/006994

(56) Entgegenhaltungen:
- EP-A1- 1 669 747
- WO-A1-2007/073111
- US-A1- 2002 184 939
- XU ET AL: "Selective detection of HCHO gas using mixed oxides of ZnO/ZnSnO3", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, Bd. 120, Nr. 2, 22. Dezember 2006 (2006-12-22), Seiten 694-699, XP005812384, ISSN: 0925-4005, DOI: 10.1016/J.SNB.2006.03.033 in der Anmeldung erwähnt
- LING ZHANG, JIFAN HU, PENG SONG, HONGWEI QIN, XIANGDONG LIU, MINHUA JIANG: "Formaldehyde-sensing characteristics of perovskite La0.68Pb0.32Fe03 nano materials", PHYSICA B: CONDENSED MATTER, Bd. 370, Nr. 1-4, 15. Dezember 2005 (2005-12-15), Seiten 259-263, XP002667762, in der Anmeldung erwähnt
- COMINI ET AL: "Metal oxide nano-crystals for gas sensing", ANALYTICA CHIMICA ACTA, ELSEVIER, AMSTERDAM, NL, Bd. 568, Nr. 1-2, 24. Mai 2006 (2006-05-24), Seiten 28-40, XP027912649, ISSN: 0003-2670 [gefunden am 2006-05-24]
- MINAMI T ET AL: "Preparation of transparent conducting In4Sn3O12 thin films by DC magnetron sputtering", THIN SOLID FILMS, ELSEVIER-SEQUOIA S.A. LAUSANNE, CH, Bd. 308-309, Nr. 1-4, 31. Oktober 1997 (1997-10-31), Seiten 13-18, XP004110237, ISSN: 0040-6090, DOI: 10.1016/S0040-6090(97)00530-0

## Beschreibung

Die vorliegende Erfindung betrifft einen Gas-Sensor zur Detektion von Gasen in der Luft, insbesondere von Formaldehyd, sowie ein Verfahren zu seiner Herstellung.

Formaldehyd ist eine chemische Verbindung, die industriell vielfältig eingesetzt wird. Es wird bei der Herstellung von Kunststoffen, bei der Holzverarbeitung als Klebstoff in Sperrholz- und Spanplatten, in der Bauindustrie als Wärmedämmung, in der Textilindustrie zur Knitterfrei- und Pflegeleicht-Ausrüstung sowie in der Landwirtschaft und Nahrungsmittelindustrie als Konservierungsmittel verwendet. Formaldehyd findet als Desinfektionsmittel Verwendung und ist darüber hinaus in Kosmetika, Körper- und Mundpflegemitteln sowie teilweise in Farben, Lacken und Teppichböden enthalten (1).

Formaldehyd entsteht außerdem bei unvollständig ablaufenden Verbrennungsprozessen. Diese finden sich beispielsweise in Verbrennungsmotoren von Kraftfahrzeugen, in Gießereien, bei der Herstellung von Kunststoffartikeln oder bei der Holzverbrennung in Kleinfeuerungsanlagen. Auch beim Rauchen entsteht auf diese Weise Formaldehyd, welches zur Belastung der Luft beiträgt (1).

Formaldehyd ist eine gasförmige Substanz, die gesundheitliche Probleme wie Augenbrennen oder Schleimhautreizungen hervorrufen kann. Es führt bei kurzfristiger Exposition bereits bei geringen Konzentrationen zu Reizungen der Augen und Atemorgane: ab 0,01 ppm Reizung der Augen, ab 0,08 ppm Reizung von Augen und Nase und ab 0,5 ppm Reizung der Kehle. Konzentrierte Dämpfe größer 10 ppm können zu schweren Reizzuständen der Schleimhäute mit Tränenfluss, Husten und Brennen in Nase und Kehle führen. Konzentrationen über 30 ppm bewirken toxisches Lungenödem und Lungenentzündung mit Lebensgefahr (1).

Chronische Wirkungen von Formaldehyd sind Befindlichkeitsstörungen wie Schlaflosigkeit, Mattigkeit, Antriebsverlust, Appetitmangel oder Nervosität, Augenreizungen und Bindehautentzündungen, Hautreizungen, chronischer Husten, Erkältungen und Bronchitis, Kopfschmerzen, Depressionen u.a. Des Weiteren kann Formaldehyd auch Allergien auslösen und steht seit einiger Zeit im Verdacht beim Menschen Krebs auslösen zu können sowie erbgutverändernd und fruchtschädigend zu wirken. Aus diesem Grund wurde vom Bundesgesundheitsamt eine maximale Arbeitsplatzkonzentration (MAK) von 0,3 ppm (0,375 mg/m³) eingeführt. Der Innenraumrichtwert liegt sogar bei nur 0,1 ppm (0,125 mg/m³), da hierbei von einer Dauerbelastung auszugehen ist (2).

Aus diesem Grund ist einer effektiven und schnellen Detektion und Messung von Formaldehyd in der Luft eine große Bedeutung zu zuschreiben.

Aus dem Stand der Technik sind einige Verfahren zur Detektion von Formaldehyd in 20 der Luft bekannt (einen Überblick über die bekannten Methoden gibt z.B. die Publikation von H. Nishikawa und T. Sakai (3).

Weitere Gassensoren, sie sich auch zur Detektion von Formaldehyd eignen, sind aus der EP 1 669 747 A1, der WO 20071073111 A1, der US 2002/184939 A1 und aus den Veröffentlichungen Comini et al.: "Metal oxide nano-crystals for gas sensing", ANALYTICA CHIMICA ACTA, ELSEVIER, AMSTERDAM, NL, Bd. 568, Nr. 1-2, 24. Mai 2006, Seiten 28-40, XP027912649, ISSN: 0003-2670 und Minami et al.: "Preparation of transparent conducting In4Sn3O12 thin films by DC magnetron sputtering", THIN SOLID FILMS, ELSEVIER-SEQUOIA S.A. LAUSANNE, CH, Bd. 308-309, Nr. 1-4, 31. Oktober 1997 (1997-10-31), Seiten 13-18, XPO04110237, ISSN: 0040-6090, bekannt.

Zu analytischen Standardmethoden gehören beispielsweise GC- und HPLC-Analysen. Das NIOSH (National Institute for Occupational Safety and Health) hat zur Abschätzung von Risiken am Arbeitsplatz einige analytische Methoden zur Detektion von Formaldehyd in der Luft standardisiert.

Bei der NIOSH-Methode 2016 wird beispielsweise die Probeluft durch ein Medium geleitet, das aus einem mit Dinitrophenylhydrazin (DNPH) beschichteten Kieselgel besteht. Die chemische Reaktion führt zu Bildung von Hydrazonen, die als stabile Derivate mit Hilfe von HPLC, GC/FID, GC/ECD oder Diodenarraydetektoren identifiziert und quantifiziert werden können (4).

Die NIOSH-Methode 2541 basiert auf GC/FID-Analysen. Hierbei wird die Probeluft durch ein mit 2-hydroxymethyl-Piperidin (2-HMP) belegtes Röhrchen geleitet. Formaldehyd aus der Probe reagiert mit 2-HMP zu einem Oxazolidin-Derivat, welches anschließend desorbiert und in einem Gaschromatograph analysiert wird (5).

Die NIOSH-Methode 3500 basiert auf spektrometrischen Messungen. Hierbei kondensiert Formaldehyd in Gegenwart von Schwefelsäure mit zwei Molekülen Chromotropsäure und es bildet sich ein rotes Carbenium-Kation. Anschließend erfolgt der spektroskopische Nachweis mittels einer Messung bei 580 nm (6).

Ein wesentlicher Nachteil der analytischen Verfahren besteht darin, dass die Luftprobe zur Derivatisierung von Formaldehyd aufwendig präpariert werden muss und die eigentliche Messung nur in einem Speziallabor durchgeführt werden kann. Eine online-Detektion ist mit diesen Methoden nicht möglich.

Neben den analytischen Methoden ist aus dem Stand der Technik eine Reihe instrumenteller Methoden bekannt. So lässt sich Formaldehyd aufgrund seines Ionisationspotentials von 10,87 eV nach einer Ionisation mit einer Argonlampe mittels eines Photoionisationsdetektors detektieren. Auch bei dieser Methode liegt der Hauptnachteil in ihrem Aufwand begründet.

Ein anderes Verfahren zur Formaldehyd-Detektion basiert auf einer elektrochemischen Zelle. Diese Methode hat den Nachteil, dass die für die Messung notwendige Apparatur sehr teuer ist. Außerdem sind für die Messgeräte regelmäßige Rekalibrierungen notwendig, wobei die Lebensdauer einer elektrischen Zelle auf weniger als ein Jahr begrenzt ist.

Des Weiteren sind aus dem Stand der Technik Fluoreszenz-basierte Verfahren zum Formaldehyd-Nachweis bekannt, z.B. eine Detektionsmethode, die auf einer Hanzsch-Reaktion basiert. Das Verfahren weist zwar eine relativ hohe Selektivität auf, das entsprechende Messgerät ist jedoch sehr teuer. Ein weiterer Nachteil besteht in einer aufwendigen Vorbereitung der Luftprobe, bei der das Formaldehyd für die Messung entsprechen derivatisiert wird (7).

Die oben genannten Verfahren zum Nachweis von Formaldehyd verlangen zur Derivatisierung und anschließender Analyse von Formaldehyd einen hohen apparativen Aufwand, so dass diese Methoden nur in großen Labors eingesetzt werden können und die Ergebnisse erst nach Ablauf von langen Präparationszeiten bekannt werden.

Es ist aus dem Stand der Technik ein MOX-basiertes Verfahren bekannt, mit dem man die Formaldehyd-Konzentrationen auch online bestimmen kann. Hierbei reagiert Formaldehyd aus der Probe mit einem Metalloxid-Sensor, der daraufhin seine Leitfähigkeit ändert. Als Sensor wird eine sensitive Schicht aus unterschiedlich kombinierten Oxiden von Zn, Ni, Sn, Cd, In und anderen Metallen verwendet. Tabelle 1 gibt einen Überblick über die bislang bekannten Metalloxide, die zur Formaldehyd-Detektion verwendet wurden, mit Angabe ihrer Messbereiche und der Autoren.

**Tabelle 1: aus dem Stand der Technik bekannte Metalloxide, die bislang zur Formaldehyd-Detektion verwendet wurden**

| **Ref.** | **Jahr** | **Autor** | **Material** | **Konzentration [ppb]** | | | **Sensorsignal [R₀/R]** | | |
|---|---|---|---|---|---|---|---|---|---|
| 8 | 2001 | Dirksen | NiO | 3,9·10⁴ | - | 8,0·10⁵ | nachweisbar | | |
| 9 | 2003 | Huang | SnO₂ | 5.0·10² | - | | 2 | - | |
| 10 | 2003 | Aronova | SnO₂: WO₃, ZnO, Pd, Pt | 1,25·10³ | - | 1,0·10⁵ | 1,6 | | |
| 11 | 2005 | Shi | IPD SnO₂ | 2,0·10¹ | - | 2,0·10² | 1,25 | - | 2,5 |
| 12 | 2005 | Zhang | La_{0,68}Pb_{0,32}FeO₃ | 1,0·10⁴ | - | 5,0·10⁵ | 2 | - | 8 |
| 13 | 2006 | Lee | NiO | 1,0·10³ | - | 1,2·10⁴ | 0 | - | 1,04 |
| 14 | 2006 | Xu | ZnO/ZnSnO₃ | 2,0·10³ | - | 5,0·10⁴ | 3 | - | 34,5 |
| 15 | 2007 | Chen | In₂O₃:CdO | 1,0·10⁴ | - | 1,0·10⁵ | 80 | - | 700 |
| 16 | 2007 | Huang | Lafe_{0,77}Zn_{0,23}O₃ | 1,0·10² | - | 5,0·10² | 44,5 | - | 188,6 |
| 17 | 2007 | Lv | SnO₂: Au, Cu, Pt or Pd | 6,0·10¹ | - | 3,0·10² | 1,03 | - | 1,17 |
| 18 | 2008 | Baia | ZnO:Ni | 1,0·10⁵ | - | | 10 | - | |
| 19 | 2008 | Chen | SnO₂-In₂O₃-CdO | 1,0·10⁴ | - | 3,0·10⁵ | 40 | - | 559 |
| 20 | 2008 | Liu | SnO₂:Sb | 1,0·10⁵ | - | 1,0·10⁶ | 2,5 | - | 6 |
| 21 | 2008 | Lv | SnO₂:NiO | 6,0·10¹ | - | 3,0·10² | 1,03 | - | 1,17 |
| 22 | 2008 | Wang | MWCNT SnO₂ | 6,0·10¹ | - | 1,0·10⁴ | 1,02 | - | 1,6 |
| 23 | 2009 | Chu | ZnO | 1,0 | - | 1.0·10⁶ | 7,4 | - | 1000 |
| 24 | 2009 | Han | ZnO:Ga | 3,2·10⁴ | - | 2,05·10⁵ | 2,3 | - | 9,8 |
| 25 | 2009 | Wang | CdIn₂O₄ | 1.0·10⁵ | - | 1,0·10⁶ | 100 | - | 800 |
| 26 | 2009 | Wang | SnO₂:Pd | 3,0·10¹ | - | 1.0·10⁴ | 1,02 | - | 1,5 |
| 27 | 2009 | Zeng | Cd-TiO2-SnO2 | 5,0·10⁴ | - | 5,0·10⁵ | 6 | - | 60 |

Aus der Tabelle 1 ist ersichtlich, dass alle bislang bekannten Gas-Sensoren, die auf der Basis von Metalloxiden funktionieren (mit Ausnahme von ZnO-Nanowires) bei sehr hohen Konzentrationsbereichen arbeiten, die weit über dem vom Gesetz festgelegten maximalen zulässigen Richtwert liegen, oder ein niedriges Sensorsignal aufweisen (Sensorsignale, die einen Konzentrationsbereich von drei Größenordnungen abdecken und lediglich in einem Bereich von 1 bis 1,6 liegen, lassen keine relevante Konzentrationsabstufung zu). Im Bezug auf die Nanowires wird in der Publikation von Chu (23) über Probleme in der Langzeitstabilität der Sensoren berichtet.

Aus US2002/0118027A1 ist ein nanostrukturiertes anodisches Aluminiumoxid-Substrat für Gas-Sensoren bekannt, das parallele Poren mit Elektroden aufweist. Das sensitive Material wird innerhalb der Poren abgeschieden, was die Oberfläche der sensitiven Schicht im Vergleich zur planar aufgetragenen Schicht stark vergrößern und die Sensitivität des Sensors somit erhöhen soll. Das für die sensitive Schicht verwendete Material spielt bei diesem Dokument eine nachgeordnete Rolle. Die Kosten für die Herstellung eines solchen Substrats dürfen relativ hoch sein.

Die Aufgabe der vorliegenden Erfindung ist es daher, einen neuen Gas-Sensor bereitzustellen, der eine hohe Sensitivität aufweist, eine online-Detektion ermöglicht und dabei preisgünstig in der Herstellung ist.

Gemäß Anspruch 1 wird diese Aufgabe durch einen Gas-Sensor gelöst, der in seinem gassensitiven Bereich das Material In₄Sn₃O₁₂ enthält. Seine bevorzugte Ausgestaltungen und Weiterbildungen, das Herstellungsverfahren sowie seine Verwendung sind Gegenstand der Unteransprüche.

Die Substanz In₄Sn₃O₁₂ ist aus dem Stand der Technik für die Verwendung bei der Herstellung von strahlungsemittierenden und elektrochromen Vorrichtungen bekannt (DE102007049005A1, DE102004001508T2, DE000060017440T2). Für die Herstellung von Sensoren ist dieses Material bislang nicht beschrieben worden.

Im Rahmen der vorliegenden Erfindung hat es sich überraschenderweise gezeigt, dass die Substanz In₄Sn₃O₁₂ Eigenschaften eines effektiven Gas-Sensors aufweist. Entscheidend für den erfindungsgemäßen Sensor ist, dass die Substanz In₄Sn₃O₁₂ als ternäres Oxid (Mischoxid-Phase) vorliegt, und nicht als eine einfache Metalloxid-Mischung. Dabei handelt es sich um ein eigenständiges Material, genauer um eine nicht-triviale Phase, der eine eigene Struktur zukommt. Diese Substanz wurde z.B. in (29) ausführlich beschrieben und charakterisiert. Dem aktuellen Stand der Technik sind keinerlei Hinweise oder Anregungen zu entnehmen, die Mischoxid-Phase In₄Sn₃O₁₂ als sensitive Schicht in einem Gas-Sensor zu verwenden.

Der erfindungsgemäße Sensor enthält mindestens einen gassensitiven Bereich, bestehend aus In₄Sn₃O₁₂, der bevorzugt als eine Schicht ausgebildet ist. Bei einer Gas-Detektion mit dem erfindungsgemäßen Sensor wird seine sensitive Schicht mit der Gasprobe (z.B. Luft) in Verbindung gebracht. Nach einer Reaktion ändern sich die elektrischen Eigenschaften der sensitiven Schicht, was sich als Änderung der elektrischen Impedanz, der Austrittsarbeit und / oder Kapazitätsänderung messen lässt. Bevorzugt wird dabei die Änderung des Widerstands gemessen.

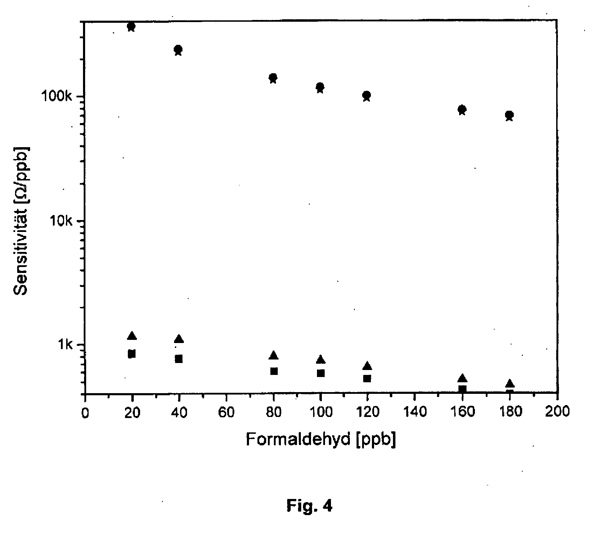

Gemäß einer bevorzugten Ausführungsform der Erfindung wird der erfindungsgemäße Sensor zur Detektion von Formaldehyd verwendet. Mit dem erfindungsgemäßen Sensor lassen sich beispielsweise für den Konzentrationsbereich von Formaldehyd zwischen 20 ppb und 180 ppb Sensorsignale von 2,1 bis 10,9 erhalten. Im Vergleich mit den kommerziell erhältlichen Referenzsensoren zeigt der erfindungsgemäße Sensor eine Steigerung des Sensorsignals von bis zu 640%. Dies entspricht einer Sensitivität, welche zwei Größenordnungen höher liegt, als die der Referenzsensoren. Während, wie in Fig. 4 gezeigt, die Sensitivität der Referenzsensoren im Bereich von 1kΩ pro ppb liegt, weißt der In₄Sn₃O₁₂-Sensor eine Sensitivität von 350kQ pro ppb auf. Ein weiterer Vorteil des erfindungsgemäßen Sensors liegt in einer geringen Sensitivität für CO: verglichen mit den kommerziell erhältlichen Sensoren beträgt das Sensorsignal für CO bei 100 ppm lediglich 19,6%.

In einer Weiterbildung der Erfindung kann der Sensor zur Detektion von anderen Gasen wie NO₂, Alkohol, CO o.a. verwendet werden.

Zum Gegenstand der Erfindung gehört ebenfalls das Verfahren zur Herstellung des erfindungsgemäßen Sensors. Dazu wird eine gassensitive In₄Sn₃O₁₂-Schicht mit Hilfe des sog. FSP-Verfahrens (Flame Spray Pyrolysis, Flammensprühpyrolyse) auf ein Substrat aufgetragen.

Das FSP-Verfahren ist aus dem Stand der Technik für die Bereitstellung von Pd/SnO₂-Sensor bekannt (L. Mädler et al., 28). Der erfinderische Schritt des vorliegenden Verfahrens gegenüber dem herkömmlichen FSP-Verfahren besteht in der Identifizierung geeigneter Ausgangssubstanzen, um eine In₄Sn₃O₁₂-Schicht herstellen zu können. Im Rahmen der vorliegenden Erfindung hat es sich herausgestellt, dass bei der Verwendung von metallorganischen Verbindungen des Indiums bzw. des Zinns als Ausgangsstoffe, gelöst in einem organischen Lösungsmittel, besonders gute Ergebnisse bei der Herstellung der sensitiven Schicht erzielt werden können. Insbesondere sind die Substanzen Indium-Acetyl-Aceton und Zinn-2-Ethyl-Hexanoat gelöst in Xylol zur Produktion von In₄Sn₃O₁₂-Schichten dafür geeignet.

Es hat sich außerdem herausgestellt, dass im Verfahren zur Herstellung der gassensitiven Schicht für den erfindungsgemäßen Sensor die Konzentrationen der Ausgangssubstanzen eine wichtige Rolle spielen. So wurden bei der Verwendung von Ausgangssubstanzen Indium-Acetyl-Aceton und Zinn-2-Ethyl-Hexanoat jeweils in der Konzentration zwischen 0,05 und 0,7 molar (mol pro Liter Lösungsmittel) die besten Ergebnisse erzielt.

Ein weiterer Gegenstand der vorliegenden Erfindung ist die Verwendung des oben beschriebenen Gas-Sensors zur Gas-Detektion im häuslichen Bereich, um eine entsprechende Belastung der Luft online analysieren zu können. Des Weiteren ist der Sensor geeignet, um eine Luftanalyse in Betrieben zu ermöglichen, welche Formaldehyd verarbeiten und deshalb eine Belastung für Mensch und Umwelt nicht ausgeschlossen werden kann.

Da es bis dato noch keine Möglichkeit gibt, Formaldehyd in einer online-Applikation nachzuweisen, stellt der erfindungsgemäße Sensor einen neuen Eckpfeiler im Bezug auf den Stand der Technik dar.

Weitere Vorteile, Merkmale und Anwendungsmöglichkeiten des Sensors und des Verfahrens zu seiner Herstellung werden nachstehend anhand der unten beschriebenen Ausführungsbeispiele mit Bezug auf die Figuren beschrieben.

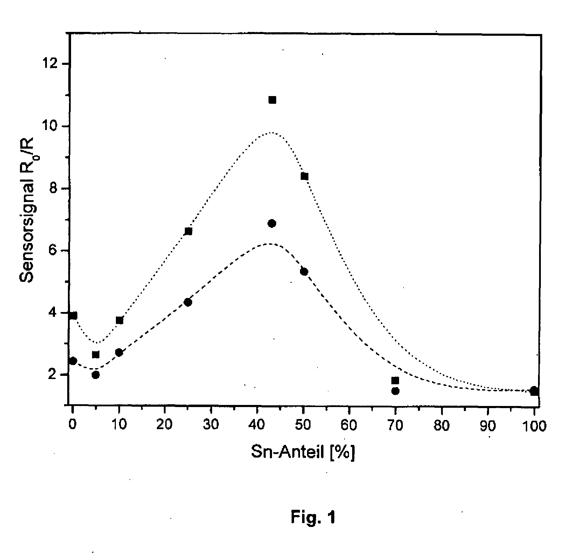

**Fig. 1****:** Gezeigt ist das Sensorsignal des erfindungsgemäßen Sensors in Abhängigkeit des Zinngehaltes. 0% entspricht reinem In₂O₃, 100% entspricht reinem SnO₂. Ein Maximum an Sensorsignal wird erreicht für einen Sn-Anteil von 43%, was der reinen Phase In₄Sn₃O₁₂ entspricht. Quadrate kennzeichnen Sensorsignale bei einer Formaldehyd Konzentration von 180ppb, Punkte kennzeichnen Sensorsignale bei einer Konzentration von 100ppb.

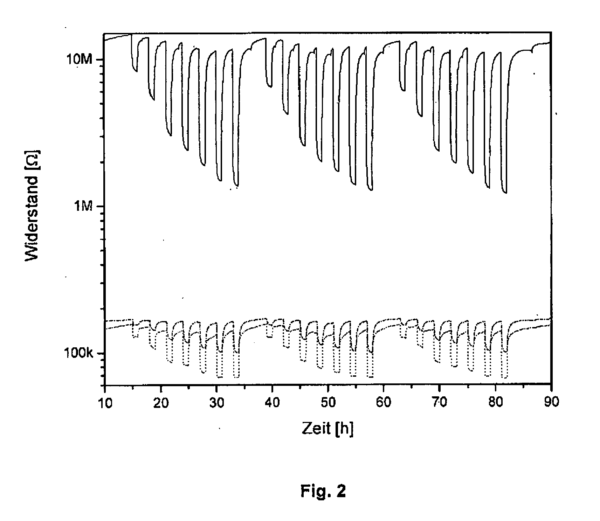

**Fig. 2****:** Gezeigt ist der Verlauf des Widerstandes bei Messungen unterschiedlicher Formaldehyd-Konzentrationen mit dem erfindungsgemäßen Sensor im Vergleich zu Messungen mit aus dem Stand der Technik bekannten Vorrichtungen über die Zeit. Die durchgezogene Linie entspricht der Messung mit dem erfindungsgemäßen Sensor mit der reinen In₄Sn₃O₁₂-Phase, die gepunktete Linie dem AppliedSensor MLC (2,3V) und die gestrichelte Line dem Figaro TGS 2620 (5,0V) Sensor. Durch die logarithmische Auftragung ist aus dem direkten, optischen Vergleich zu erkennen, dass das Sensorsignal für den erfindungsgemäßen Sensor deutlich größer ist, als das der aus dem Stand der Technik bekannten Sensoren. Die den einzelnen Signalstufen zuzuordnenden Konzentrationen betragen 20, 40, 80, 100, 120, 160 und 180 ppb, dann wiederholt sich die Sequenz.

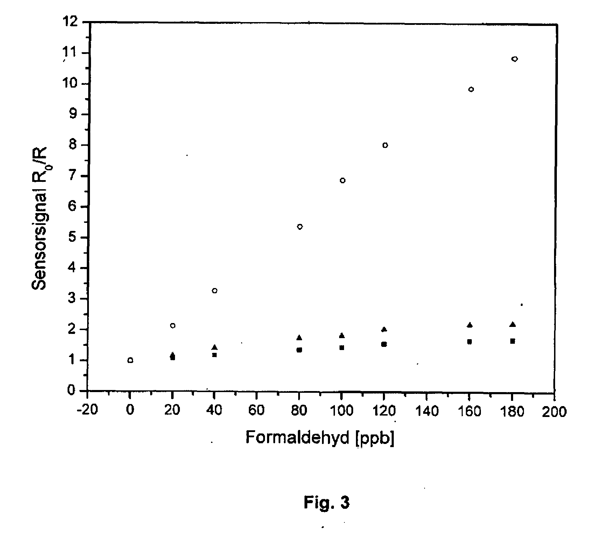

**Fig. 3****:** Gezeigt ist der Verlauf des Sensorsignals bei unterschiedlichen Konzentrationen von Formaldehyd in feuchter Luft (50% Rel. Feuchte). Klar erkennbar ist das gesteigerte Sensorsignal der In₄Sn₂O₁₂-Phase (Kreise) gegenüber den Sensorsignalen, gemessen mit den aus dem Stand der Technik bekannten Referenzsensoren von Figaro (Quadrate) und AppliedSensor (Dreiecke) in jedem Konzentrationsbereich. Beide aus dem Stand der Technik bekannten Referenzsensoren arbeiten wie der erfindungsgemäße Sensor auf Basis einer Widerstandsänderung, allerdings basiert ihre sensitive Schicht auf Zinndioxid.
**Fig. 4****:** Gezeigt ist die Sensitivität des erfindungsgemäßen Sensors im Vergleich mit den zwei aus dem Stand der Technik bekannten Referenzsensoren. Quadrate entsprechen dem Figaro TGS 2620, Dreiecke entsprechen dem AppliedSensor MLC und Punkte bzw. Sterne zeigen die Sensitivität des erfindungsgemäßen In₄Sn₃O₁₂-Sensors an zwei unterschiedlichen Tagen. Gemäß der Definition von Sensitivität wird hierbei die Änderung des Widerstandes relativ zur Änderung der Analytkonzentration gegen die Analytkonzentration aufgetragen. Deutlich zu erkennen ist, dass der erfindungsgemäße Sensor eine Sensitivität aufweist, die zwei Größenordnungen höher liegt, als die Sensitivität der aus dem Stand der Technik bekannten Referenzsensoren.

### Ausführungsbeispiele

### Darstellung des Materials und Abscheidung auf dem Sensorsubstrat

In Anbetracht des Phasendiagramms der festen Lösung von SnO₂ in In₂O₃ (I. Isomäki et al. (29)) ist erkennbar, dass es sich bei der Phase In₄Sn₃O₁₂ um eine metastabile Hochtemperaturphase handelt, die sich im Temperaturbereich von rund 1600 bis 1900 K bildet. Erniedrigt man die Temperatur langsam (geht man im Diagram vertikal nach unten), so zerfällt die Phase in eine feste Lösung von ITO und SnO₂. Bei Kompositionen mit einem Sn-Anteil von mehr als 10%, lässt sich, durch die geeignete Wahl der Temperatur, stets die In₄Sn₃O₁₂ Phase darstellen. Geht man mit der Temperatur noch höher, so bildet sich schlussendlich eine ionische Flüssigkeit. Die Synthesemethode der Flammensprühpyrolyse ermöglicht somit die Darstellung der Phase in der Flamme und die Abscheidung auf ein gekühltes Substrat stellt sicher, dass diese Phase abgeschreckt und somit erhalten bleibt.

Organometallische Verbindungen wie Indiumacetylaceton (99,9% rein, Strem) oder Zinn-2-Ethylhexanoat (99,5% rein, Strem) wurden in der Flammensprühpyrolyse (FSP) verwendet, um Zinn dotierte In₂O₃ (ITO) Metalloxide herzustellen. Die Organometallverbindungen, nachfolgend Precursoren, wurden in organischen Lösungsmitteln (beispielsweise Toluol (99,95% rein, Strem) oder Xylol (99,99% rein, Sigma Aldrich) gelöst, um Konzentration von 0,15M zu erhalten. Als Standartparameter während der Synthese wurde ein Volumenstrom des Precursors von 5 ml/min gewählt. Zerstäubt wurden die Lösungen mittels einer Düse und einem parallel einströmenden Sauerstoffvolumenstrom von 5 l/min, bei einem Zerstäubungsdruck von 1,6 bar an der Düse. Die Verbrennung des Precursor-Sprays wurde injiziert durch eine kreisförmige Methan / Sauerstoff-Flamme (1,5 l/min 3,2 l/min).

Die Zusammensetzung der synthetisierten Phase lässt sich aus Tabelle 2 entnehmen. Je nach Verhältnis der eingesetzten Precursoren lassen sich so gezielt Zusammensetzungen der sensitiven Schicht erhalten. Man erkennt aus der Tabelle, dass bei einer Zinnkonzentration von 43% die reine In₄Sn₃O₁₂-Phase vorliegt.

Die Sensorsubstrate (Hereaus) wurden in einem Abstand von 25 cm über der Flamme platziert und rückseitig durch einen entsprechenden Probenhalter wassergekühlt. Abscheidungsdauer belief sich auf 20 min.

**Tabelle 2: Messwerte angestrebter Zinnkonzentrationen und die Zusammensetzung des erhaltenen Materials.**

| Sn / (Sn+In) [%] | Massen-% von SnO₂ bzw. ITO | | Massen-% von In₄Sn₃O₁₂ | |
|---|---|---|---|---|
| Nominal | Erwartet | Gefunden (XRD) | Erwartet | Gefunden (XRD) |
| 0 | 100 | Nicht gemessen | 0 | Nicht gemessen |
| 5 | 100 | 100 | 0 | 0 |
| 10 | 89 | 96 | 11 | 4 |
| 17 | 70 | 77 | 30 | 23 |
| 25 | 48 | 49 | 52 | 51 |
| 43 | 0 | 0 | 100 | 100 |
| 50 | | 2 | | 98 |
| 60 | | 6 | | 94 |
| 70 | | 47 | | 53 |
| 80 | | 93 | | 7 |
| 100 | 100 | 100 | 0 | 0 |

### Messung des Widerstandes und Temperaturkalibrierung

Die Substrate werden in einem Ofen erhitzt und der Widerstand der rückseitigen Heizwendel bestimmt. Die entstehende Kalibrierkurve wird als Basis für den Betrieb des Sensors genutzt.

Die Sensoren werden auf entsprechende Messkammern eingetragen, welche an eine spezielle, extra für das Arbeiten mit kleinen Konzentrationen von Formaldehyd entwickelte, Gasmischanlage (Röck et al. (30)) angeschlossen wird. Der Widerstand der Sensitiven Schicht wird durch ein Multimeter (Agilent 34970A) ausgelesen, welches in Kombination mit einem Rechner die Aufnahme der Messdaten gewährleist. **Figur 2** zeigt den Verlauf einer Widerstandsmessung über die Zeit. Diese Daten können durch mathematische Operationen in die Größen Sensorsignal und Sensitivität umgewandelt werden, um einen Anhaltspunkt über die Qualität eines Sensors zu einer bestimmten Anwendung zu erhalten. In **Figur 1** sind die Sensorsignale der unterschiedlichen Kompositionen der sensitiven Schichten aufgezeigt. Diese Daten lassen sich, für eine Komposition mit 43% Sn-Anteil, direkt aus dem in **Figur 3** gezeigten Verlauf des Sensorsignals entnehmen.

### Referenzen

1. WHO Regional Office for Europe, Copenhagen, 2001, http://test.cp.euro.who.int/document/aiq/5 8formaldehyde.pdf
2. Bundesinstitut für Risikobewertung, Toxikologische Bewertung von Formaldehyd vom 30.03.2006, http://www.bfr.bund.de/cm/252/toxikologische bewertung von formaldehyd.pdf
3. H.Nishikawa, T. Sakai, Derivatization and Chromatographic Determination of Aldehydes in Gaseous and Air Samples, Journal of Chromatography A, vol. 710, pp. 159-165, 1995.
4. NIOSH Manual of Analytical Methods, Formaldehyde: Method 2016, 4th Edition, 2003, http://www.cdc.gov/niosh/docs/2003-154/pdfs/2016.pdf
5. NIOSH Manual of Analytical Methods, Formaldehyde: Method 2541, 4th Edition, 1994, http://www.cdc.gov/niosh/docs/2003-154/pdfs12541.pdf
6. NIOSH Manual of Analytical Methods, Formaldehyde: Method 3500, 4th Edition, 1994, http://www.cdc.gov/niosh/docs/2003-154/pdfs/3500.pdf
7. VdL RL-03, VdL-Richtlinie Formaldehydbestimmung, 1997, http://www.lackindustrie.de/template downloads/tmp LackverbandInternet/79 811VDLRL03-597.pdf?DokNr=79811&p=16
8. James A. Dirksen, Kristin Duval, Terry A. Ring, NiO thin-film formaldehyde gas sensor, Sensors and Actuators B: Chemical, Volume 80, Issue 2, 20 November 2001, Pages 106-115, ISSN 0925-4005, DOI: 10.1016/S0925-4005(01)00898-X.
9. Xingjiu Huang, Fanli Meng, Zongxin Pi, Weihong Xu, Jinhuai Liu, Gas sensing behavior of a single tin dioxide sensor under dynamic temperature modulation, Sensors and Actuators B: Chemical, Volume 99, Issues 2-3, 1 May 2004, Pages 444-450, ISSN 0925-4005, DOI: 10.1016/j.snb.2003.12.013.
10. Liqin Shi, Wei Gao, Yuki Hasegawa, Teruaki Katsube, Mamoru Nakano, Kiyozumi Nakamura, High Sensitive Formaldehyde Gas Sensor Prepared by R.F. Induction Plasma Deposition Method. IEEJ Transactions on Sensors and Micromachines, 2005. 125(12): p. 485-489.
11. Ling Zhang, Jifan Hu, Peng Song, Hongwei Qin, Xiangdong Liu, Minhua Jiang, Formaldehyde-sensing characteristics of perovskite La0.68Pb0.32FeO3 nanomaterials, Physica B: Condensed Matter, Volume 370, Issues 1-4, 15 December 2005, Pages 259-263.
12. Chia-Yen Lee, Che-Ming Chiang, Yu-Hsiang Wang, Rong-Hua Ma, A selfheating gas sensor with integrated NiO thin-film for formaldehyde detection, Sensors and Actuators B: Chemical, Volume 122, Issue 2, 26 March 2007, Pages 503-510, ISSN 0925-4005, DOI: 10.1016/j.snb.2006.06.018.
13. Jiaqiang Xu, Xiaohua Jia, Xiangdong Lou, Guoxi Xi, Jianjun Han, Qiaohuan Gao, Selective detection of HCHO gas using mixed oxides of ZnO/ZnSnO3, Sensors and Actuators B: Chemical, Volume 120, Issue 2, 10 January 2007, Pages 694-699, ISSN 0925-4005, DOI: 10.1016/j.snb.2006.03.033.
14. T. Chen, Q.J. Liu, Z.L. Zhou, Y.D. Wang, The fabrication and gas-sensing characteristics of the formaldehyde gas sensors with high sensitivity, Sensors and Actuators B: Chemical, Volume 131, Issue 1, Special Issue: Selected Papers from the 12th International Symposium on Olfaction and Electronic Noses - ISOEN 2007, International Symposium on Olfaction and Electronic Noses, 14 April 2008, Pages 301-305, ISSN 0925-4005, DOI: 10.1016/j.snb.2007.11.025.
15. Shanxing Huang, Hongwei Qin, Peng Song, Xing Liu, Lun Li, Rui Zhang, Jifan Hu, Hongdan Yan, Minhua Jiang, The formaldehyde sensitivity of LaFe1-x Zn x O3-based gas sensor. Journal of Materials Science, 2007. 42(24): p. 9973-9977.
16. Pin Lv, Zhenan Tang, Guangfen Wei, Jun Yu, Zhengxing Huang, Recognizing indoor formaldehyde in binary gas mixtures with a micro gas sensor array and a neural network. Measurement Science and Technology, 2007. 18(9): p. 2997.
17. Zikui Bai, Changsheng Xie, Mulin Hu, Shunping Zhang, Formaldehyde sensor based on Ni-doped tetrapod-shaped ZnO nanopowder induced by external magnetic field. Physica E: Low-dimensional Systems and Nanostructures, 2008. 41(2): p. 235-239.
18. T. Chen , Q. J. Liu , Z. L. Zhou, Y. D. Wang, A high sensitivity gas sensor for formaldehyde based on CdO and In 2 O 3 doped nanocrystalline SnO 2. Nanotechnology, 2008. 19(9): p. 095506.
19. Jinyun Liu, Zheng Guo, Fanli Meng, Yong Jia, Jinhuai Liu, A Novel Antimony-Carbon Nanotube-Tin Oxide Thin Film: Carbon Nanotubes as Growth Guider and Energy Buffer. Application for Indoor Air Pollutants Gas Sensor. The Journal of Physical Chemistry C, 2008. 112(15): p. 6119-6125.
20. Pin Lv, Zhen A. Tang, Jun Yu, Feng T. Zhang, Guang F. Wei, Zheng X. Huang, Yann Hu, Study on a micro-gas sensor with SnO2-NiO sensitive film for indoor formaldehyde detection. Sensors and Actuators B: Chemical, 2008. 132(1): p. 74-80.
21. Jing Wang, Li Liu, Song-Ying Cong, Jin-Qing Qi, Bao-Kun Xu, An enrichment method to detect low concentration formaldehyde. Sensors and Actuators B: Chemical, 2008. 134(2): p. 1010-1015.
22. Xiangfeng Chu, Tongyun Chen, Wangbing Zhang, Banqiao Zheng, Hengfu Shui, Investigation on formaldehyde gas sensor with ZnO thick film prepared through microwave heating method. Sensors and Actuators B: Chemical, 2009. 142(1):p. 49-54.
23. Ning Han, Yajun Tian, Xiaofeng Wu, Yunfa Chen, Improving humidity selectivity in formaldehyde gas sensing by a two-sensor array made of Gadoped ZnO. Sensors and Actuators B: Chemical, 2009. 138(1): p. 228-235.
24. Yude Wang, Ting Chen, Qiuying Mu, Guofeng Wang, A nonaqueous sol-gel route to synthesize CdIn2O4 nanoparticles for the improvement of formaldehyde-sensing performance, Scripta Materialia, Volume 61, Issue 10, November 2009, Pages 935-938, ISSN 1359-6462, DOI: 10.1016/j.scriptamat.2009.07.029.
25. Jing Wang, Peng Zhang, Jin-Qing Qi, Peng-Jun Yao, Silicon-based micro-gas sensors for detecting formaldehyde, Sensors and Actuators B: Chemical, Volume 136, Issue 2, 2 March 2009, Pages 399-404, ISSN 0925-4005, DOI: 10.1016/j.snb.2008.12.056.
26. Zeng W., Liu T., Wang Z., Tsukimoto S., Saito M., Ikuhara Y. Selective Detection of Formaldehyde Gas Using a Cd-Doped TiO2-SnO2 Sensor. Sensors. 2009; 9(11):9029-9038.
27. M. A. Aronova, K. S. Chang, I. Takeuchi, H. Jabs, D. Westerheim, A. Gonzalez-Martin, J. Kim, B. Lewis, Combinatorial libraries of semiconductor gas sensors as inorganic electronic noses - Appl. Phys. Lett. 83, 6, 1255-1257.
28. L. Mädler, A. Rössler, S.E. Pratsinis, T. Sahm, A. Gurlo., N. Barsan, U. Weimar. Sensors and Actuators B 114 (2006) 283-295.
29. I. Isomäki, M.H., W. Gierlotka, B. Onderka, K. Fitzner, Thermodynamic evaluation of the In-Sn-O system. Journal of Alloys and Compounds, 2006(422): p. 173-177.
30. F. Röck, N. Barsan, U. Weimar, vorgelegt bei "Measurement Science and Technology", 2010.

## Patentansprüche

1. Sensor zur Detektion von Gasen, aufweisend mindestens einen gassensitiven Bereich, der auf einem Substrat aufgebracht ist, **dadurch gekennzeichnet, dass** der gassensitive Bereich die ternäre Verbindung In₄Sn₃O₁₂ enthält.

2. Sensor nach Anspruch 1, **dadurch gekennzeichnet, dass** der mindestens eine gassensitive Bereich als eine Schicht ausgebildet ist.

3. Sensor nach einem der vorhergehenden Ansprüchen, **dadurch gekennzeichnet, dass** der mindestens eine gassensitive Bereich im Flammensprühpyrolyse-Verfahren (FSP) aufgebraucht ist.

4. Verfahren zur Herstellung eines Sensors gemäß einem der Ansprüche 1 bis 3, bei dem die Herstellung des gassensitiven Bereichs mittels einer Flammensprühpyrolyse (FSP) erfolgt.

5. Verfahren nach Anspruch 4, **dadurch gekennzeichnet, dass** als Ausgangsstoffe metallorganische Verbindungen des Indiums und des Zinns, gelöst in einem organischen Lösungsmittel, verwendet werden.

6. Verfahren nach einem der Ansprüche 4 bis 5, **dadurch gekennzeichnet, dass** die Ausgangsstoffe Indium-Acetyl-Aceton und Zinn-2-Ethyl-Hexanoat sind.

7. Verfahren nach Anspruch 6, **dadurch gekennzeichnet, dass** die Ausgangsstoffe Indium-Acetyl-Aceton und Zinn-2-Ethyl-Hexanoat jeweils in der gleichen Konzentration zwischen 0,05 und 0,7 molar eingesetzt werden.

8. Verwendung des Sensors gemäß einem der Ansprüche 1 bis 3 zur Online-Gasdetektion.

9. Verwendung des Sensors gemäß einem der Ansprüche 1 bis 3 zur Detektion von Formaldehyd.

10. Verwendung des Sensors gemäß einem der Ansprüche 1 bis 3 zu Gasdetektion im häuslichen Bereich oder in Betrieben.

## Claims

1. A sensor for detecting gases, comprising at least one gas-sensitive zone applied on a substrate, **characterized in that** the gas-sensitive zone contains the ternary compound In₄Sn₃O₁₂.

2. The sensor according to claim 1, **characterized in that** the at least one gas-sensitive zone is in the form of a layer.

3. The sensor according to any of the preceding claims, **characterized in that** the at least one gas-sensitive zone is applied using a flame spray pyrolysis method (FSP).

4. A method for producing a sensor according to any of the claims 1 to 3 wherein the production of the gas-sensitive zone is effected by means of a flame spray pyrolysis (FSP).

5. The method according to claim 4, **characterized in that** organometallic compounds of indium and tin, dissolved in an organic solvent, are used as source materials.

6. The method according to any of the claims 4 to 5, **characterized in that** the source materials are indium acetylacetone or tin-2-ethylhexanoate.

7. The method according to claim 6, **characterized in that** the source materials indium acetylacetone or tin-2-ethylhexanoate are in each case used in the same concentrations between 0.05 and 0.7 mol.

8. Use of the sensor according to any of the claims 1 to 3 for online gas detection.

9. Use of the sensor according to any of the claims 1 to 3 for detecting formaldehyde.

10. Use of the sensor according to any of the claims 1 to 3 for detecting gas in the home environment or in business establishments.

## Revendications

1. Capteur de détection de gaz, présentant au moins une zone sensible au gaz appliquée sur un substrat, **caractérisé en ce que**
la zone sensible au gaz contient le composé ternaire In₄Sn₃O₁₂.

2. Capteur selon la revendication 1, **caractérisé en ce que** la ou les zones sensibles au gaz sont configurées en couches.

3. Capteur selon l'une des revendications précédentes, **caractérisé en ce que** la ou les zones sensibles au gaz sont appliquées par une opération de pyrolyse à pulvérisation à la flamme (FSP).

4. Procédé de fabrication d'un capteur selon l'une des revendications 1 à 3, dans lequel la zone sensible au gaz est réalisée par pyrolyse à pulvérisation à la flamme (FSP).

5. Procédé selon la revendication 4, **caractérisé en ce qu'**il utilise comme matières de départ des composés organométalliques de l'indium et de l'étain dissous dans un solvant organique.

6. Procédé selon l'une des revendications 4 à 5, **caractérisé en ce que** les substances de départ sont l'indium acétylacétone et le 2-éthylhexanoate d'étain.

7. Procédé selon la revendication 6, **caractérisé en ce que** les substances de départ indium acétylacétone et 2-éthylhexanoate d'étain sont utilisées à une même concentration molaire comprise entre 0,05 et 0,7.

8. Utilisation du capteur selon l'une des revendications 1 à 3 pour la détection de gaz en ligne.

9. Utilisation du capteur selon l'une des revendications 1 à 3 pour la détection du formaldéhyde.

10. Utilisation du capteur selon l'une des revendications 1 à 3 pour la détection de gaz dans un environnement domestique ou dans des entreprises.
